# EUROPEAN PATENT APPLICATION

(11) **EP 0 801 949 A1**
(43) Date of publication of application: **22.10.1997**
(21) Application number: 97105951.4
(22) Date of filing: 10.04.1997
(51) Int. Cl.: A61K 31/19, A61K 31/20

(54) **Hypoglycemic quinone derivative**

(30) Priority: 15.04.1996 JP 92378/96
(71) Applicant: Eisai Co., Ltd., Tokyo (JP)
(72) Inventor: Yamanaka, Takashi, Tsukuba-shi, Ibaraki (JP); Ito, Kaori, Nerima-ku, Tokyo (JP); Yamatsu, Isao, Ushiku-shi, Ibaraki (JP)
(74) Representative: Herrmann-Trentepohl, Werner, Dipl.-Ing.

(57) **Abstract**

To provide a hypoglycemic agent and a diabetes preventing, curing or ameliorating agent, which exert excellent pharmaceutical effects.

A hypoglycemic agent and a diabetes preventing, curving or ameliorating agent, each of which comprises as an active ingredient a quinone derivative represented by the following general formula (I) or pharmacologically acceptable salts thereof: 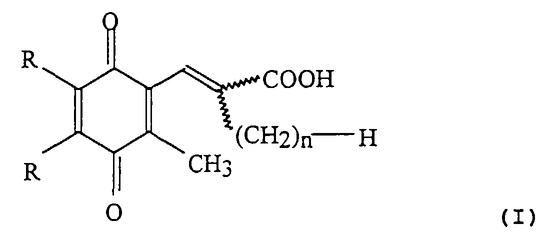 wherein n is 0 or an integer of 1 to 12 and R is a methyl or methoxy group.

## Description

### Background of Invention

The present invention relates to a hypoglycemic agent and a diabetes preventing, curing or ameliorating agent which exert excellent pharmaceutical effects.

The morbidity of adult diseases is increasing in accordance with the aging of population, the improvement of living standard, the change of meal and life style, etc. Above all, the increase of diabetes cases is serious. With respect to the diabetes, there exists one of the juvenile onset type whose prognosis is worse than that of the diabetes of the adult disease type, thereby necessitating strict treatment, although the number of cases of the former type is smaller than that of the latter type.

Moreover, diabetes patients highly frequently suffer from complications such as arteriosclerosis, hypertension, hyperlipemia, hyperuricemia, nephropathy, retinopathy and neuropathy, which would often combine to thereby cause death.

### Prior Art

In the treatment of diabetes, the diet and exercise therapy and the improvement of life style such as limitation of alcohol drinking constitute important factors. However, drug therapy is essential for attaining satisfactory effect. Commonly employed therapeutic agents are insulin, sulfonylureas and biguanides. Recently, carbohydrate absorption inhibitors have been put on the market, and insulin resistance ameliorators and insulin secretion stimulating agents are now being developed.

However, there still remain many unclear points with respect to the mechanism of onset of diabetes and the current situation is that there is no radical method of treatment. Thus, it is needed to control the blood sugar through life. In this connection, insulin is an agent given by injection, so that it is not suitable for long-term administration. Further, insulin is not effective in the treatment of noninsulin-dependent diabetes melitus (NIDDM) which is characterized by a prolonged duration of the state of high blood sugar level after meal although the fasting blood sugar level is not so high before meal and between meals.

The other drugs are orally given, so that there is no problem on the easiness of taking them for a prolonged period of time. However, the most widely employed sulfonylureas (for example, tolbutamide, glibenclamide and gliclazide) may induce severe delayed hypoglycemia as a side effect and they have involved the problem that it is difficult to control the blood sugar at an appropriate level.

Biguanides (for example, buformin hydrochloride and metformin hydrochloride) may also induce severe lactic acidosis or hypoglycemia as a side effect and, hence, their use is limited to cases in which the effect of sulfonylureas is poor or in which the use of sulfonylureas is not suitable because of the side effects.

Furthermore, the hypoglycemic effect of carbohydrate absorption inhibitors (acarbose) is so poor that their use is limited to supplemental therapy made when any satisfactory blood sugar control cannot be attained by diet and exercise therapy.

Insulin resistance ameliorators (for example, AD-4833 and CS-045) and insulin secretion stimulating agents (for example, glimepiride) have not yet been marketed, and their pharmaceutical efficacy and safety have not yet been fully evaluated.

As apparent from the above, the conventional therapeutic agents for diabetes have drawbacks such as unfavorable side effects and the current situation is that there is no radical method of treatment.

### Detailed description

Therefore, the inventors have extensively and intensively studied a variety of compounds for years with a view toward developing a therapeutic agent for diabetes which can be orally administered and is highly safe. As a result, it has been found that, unexpectedly, the below described quinone derivative represented by the general formula (I) can attain the intended object and the present invention has been completed. The quinone derivative (I) of the present invention is characterized by having both excellent hypoglycemic effect and safety and, hence, the value of the present invention is very high.

The present invention provides a method for lowering blood sugar or glucose, comprising administering a pharmacologically effective amount of a quinone derivative represented by the following general formula (I) or pharmacologically acceptable salts thereof to a person suffering from the hypoglycemic. 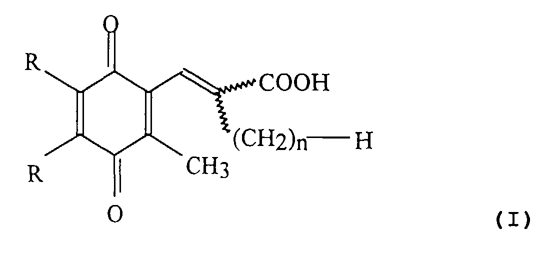 wherein n is 0 or an integer of 1 to 12 and R is a methyl or methoxy group.

The present invention further provides a method for preventing, curing or ameliorating diabetes, comprising administering a pharmacologically effective amount of a quinone derivative represented by the above shown general formula (I) or pharmacologically acceptable salts thereof to a person suffering from the diabetes.

The present invention further provides both above methods, wherein the quinone derivative (I) is (E)-3-[2-(5,6-dimethoxy-3-methyl-1,4-benzoquinonyl)]-2 -nonylpropenoic acid represented by the following chemical formula (II):

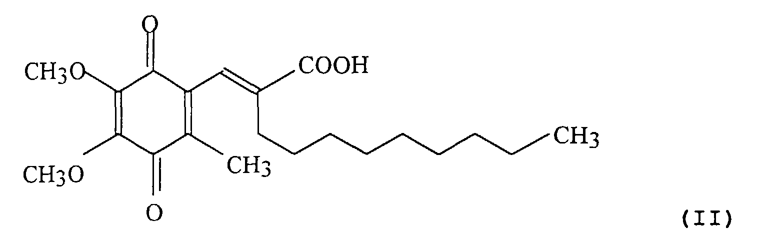

The present invention provides a use of a quinone derivative represented by the above shown general formula (I) or pharmacologically acceptable salts thereof for manufacturing a hypoglycemic agent.

The present invention further provides use of a quinone derivative represented by the above shown general formula (I) or pharmacologically acceptable salts thereof for manufacturing a diabetes preventing, curing or ameliorating agent.

The quinone derivative (I) of the present invention is represented by the following general formula:

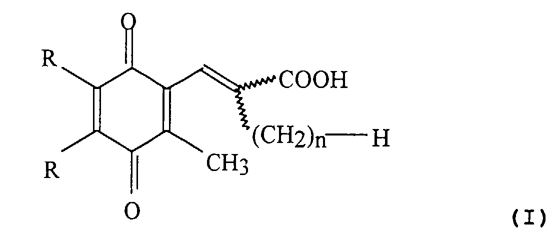

In the formula, n is 0 or an integer of 1 to 12 and R is a methyl or methoxy group.

The quinone derivative (I) of the present invention has double bonds in its molecule, so that there are geometrical isomers. The geometrical isomers are not limited in the present invention, and the quinone derivative (I) may be in the form of either a single isomer (E-isomer or Z-isomer) selected from among the isomers or a mixture of two or more of isomers (EZ mixture). Moreover, the quinone derivative (I) may be formed into a hydrate.

The pharmacologically acceptable salts of the present invention are not limited as long as they are in the form of an addition salt of the quinone derivative (I) of the present invention. Examples thereof include alkali metal addition salts such as sodium, potassium and lithium salts, alkaline earth metal addition salts such as magnesium and calcium salts, amine addition salts and amino acid addition salts.

The following compounds can be mentioned as specific examples of the quinone derivatives (I), to which the present invention is not limited.
(1) 3-[2-(5,6-dimethoxy-3-methyl-1,4-benzoquinonyl)]propenoic acid;
(2) 3-[2-(5,6-dimethoxy-3-methyl-1,4-benzoquinonyl)]-2-propylpropenoic acid;
(3) 3-[2-(5,6-dimethoxy-3-methyl-1,4-benzoquinonyl)]-2-hexylpropenoic acid;
(4) 3-[2-(5,6-dimethoxy-3-methyl-1,4-benzoquinonyl)]-2-nonylpropenoic acid;
(5) 3-[2-(5,6-dimethoxy-3-methyl-1,4-benzoquinonyl)]-2-dodecylpropenoic acid;
(6) 3-[2-(3,5,6-trimethyl-1,4-benzoquinonyl)]propenoic acid;
(7) 3-[2-(3,5,6-trimethyl-1,4-benzoquinonyl)]-2-propylpropenoic acid;
(8) 3-[2-(3,5,6-trimethyl-1,4-benzoquinonyl)]-2-hexylpropenoic acid;
(9) 3-[2-(3,5,6-trimethyl-1,4-benzoquinonyl)]-2-nonylpropenoic acid; and
(10) 3-[2-(3,5,6-trimethyl-1,4-benzoquinonyl)]-2-dodecylpropenoic acid.

Of these quinone derivatives (I), 3-[2-(5,6-dimethoxy-3-ethyl-1,4-benzoquinonyl)]-2-nonylpropenoic acid is preferred, and (E)-3-[2-(5,6-dimethoxy-3-methyl-1,4-benzoquinonyl)]-2-nonylpropenoic acid represented by the following chemical formula (II) is still preferred:

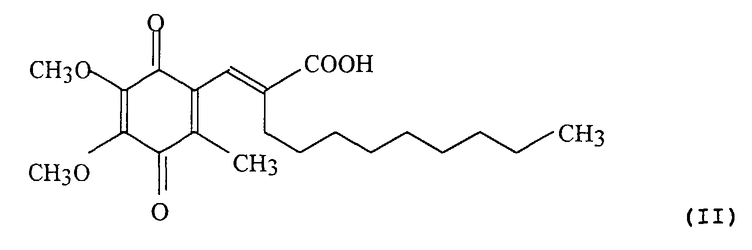

The quinone derivative (I) of the present invention can be prepared by the process described in EP-A 419905.

Dosage forms of the compound of the present invention are, for example, oral preparations such as powders, fine granules, granules, tablets, coated tablets and capsules, external preparations such as ointments and patches, suppositories and injections. Such pharmaceutical preparations can be prepared with the use of commonly accepted carriers for pharmaceutical preparations in accordance with the customary procedure.

Specifically, for producing a oral preparation, the compound of the present invention is blended with a additive and, if necessary, an antioxidant, a binder, a disintegrator, a lubricant, a coloring agent, a corrigent, etc., followed by formation of powders, fine subtilaes, granules, tablets, coated tablets or capsules in accordance with the customary procedure.

Examples of the additives include lactose, corn starch, sucrose, glucose, mannitol, sorbitol, crystalline cellulose and silicon dioxide. Examples of the binders include polyvinyl alcohol, polyvinyl ether, methylcellulose, ethylcellulose, acacia, tragacanth, gelatin, shellac, hydroxypropylmethylcellulose, hydroxypropylcellulose, polyvinylpyrrolidone, polypropylene glycol/polyoxyethylene block copolymer and meglumine. Examples of the disintegrators include starch, agar, gelatin powder, crystalline cellulose, calcium carbonate, sodium bicarbonate, calcium citrate, dextrin, pectin and calcium carboxymethylcellulose. Examples of the lubricants include magnesium stearate, talc, polyethylene glycol, silica and hydrogenated vegetable oil. Any colorant of which the addition to pharmaceuticals is officially allowed can be used as the colorant. Examples of the corrigents include cacao powder, menthol, aromatic powder, mentha oil, borneol and powdered cinnamon bark. It is a matter of course that a sugar coating and, if necessary, suitable other coatings may be applied on these tablets and granules.

For producing an injection, the compound of the present invention may be used together with a pH modifier, a solubilizing agent, a tonicity agent, etc. and, if necessary, a solubilizing auxiliary, a stabilizer, an antioxidant, etc., followed by the processing into an injection form in accordance with the customary procedure.

The process for producing external preparations is not limited, and they can be produced by conventional methods. In the production of external preparations, various materials customarily employed in pharmaceutical preparations, quasi-drugs, cosmetics, etc. can be used as the base material.

Examples of the employed base materials include animal and vegetable oils, mineral oil, ester oil, waxes, higher alcohols, fatty acids, silicone oil, surfactants, phospholipids, alcohols, polyhydric alcohols, water soluble polymers, clay minerals and purified water. Further, according to necessity, a pH modifier, an antioxidant, a chelating agent, an antiseptic/fungicide, a colorant, a perfume, etc. can be added to the above, though the base materials of the external preparations of the present invention are not limited to these materials. Still further, according to necessity, a blood flow promoter, a bactericide, an antiinflammatory agent, a cell activator, vitamins, an amino acid, a humectant, a keratolytic agent and other ingredients can be added to the external preparations. Each of the above base materials is added in such an amount that the concentration to be set in the production of common external preparations is realized.

The clinical dose of the quinone derivative (I) of the present invention or pharmacologically acceptable salts thereof is not limited and varies depending on the symptom, severity, age and complication and further depending on the type of salt, route of administration, etc. However, in general, the daily dose ranges from 0.01 to 2000 mg, preferably, from 0.1 to 1000 mg and, still preferably, from 1 to 500 mg per adult, which is given orally, intravenously, intramuscularly, per rectum or percutaneously.

### Technical Advantages

The efficacy of the compound of the present invention as a hypoglycemic agent or a diabetes preventing, curing or ameliorating agent will be demonstrated with reference to the following Pharmacological Experiment and Toxicity Test Examples, which in no way limit the scope of the compounds of the present invention and the use thereof.

### Brief Description of the Drawing

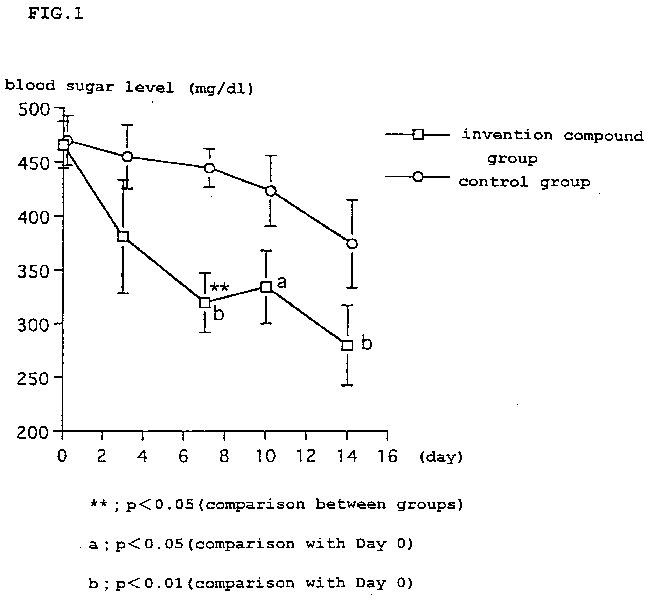

Fig. 1 is a graph showing the change of blood sugar level of each of the group dosed with the compound of the present invention and the control group. (average ± standard error).

### Examples

### Pharmacological Experiment Example

### (Experimental Method 1)

Use was made of 22-week old male KK-Ay/Ta mice (type-II diabetes model mice available from Clea Japan Inc.). Restricted feeding was effected such that the animals were starved from 9:00 a.m. to 5:00 p.m. daily through the experimental period.

(E)-3-[2-(5,6-Dimethoxy-3-methyl-1,4-benzoquinonyl)]-2-nonylpropenoic acid as a representative example of the compounds of the present invention was suspended in 0.5% methylcellulose (MC) and orally administered twice 9:00 a.m. and 5:00 p.m. daily for 14 days in accordance with the following schedule.

The 0.5% methylcellulose (MC) was administered to a control group in the same manner as above.

**Table 1**

| | Dose (mg/Kg/day) | Given a.m. (mg/Kg) | Given p.m. (mg/Kg) | No. of animals (N=) |
|---|---|---|---|---|
| control group (MC) | 0 | 0 | 0 | 6 |
| test compd. | 300 | 100 | 200 | 6 |

All blood specimens were collected from the cardal veins, and blood sugar and blood insulin levels were measured on the day preceding the administration (day 0) and 3 days (day 3), 7 days (day 7), 10 days (day 10) and 14 days (day 14) after the administration. Hemoglobin AIc (HbAIc) was measured on day 0 and day 14. The measurements of blood sugar level, blood insulin level and HbAIc were conducted by the use of Fuji Dry-Chem System 5500 (manufactured by Fuji Medical Systems Co., Ltd.), insulin measuring kit (EIA sandwich method, manufactured by Morinaga Seikagaku Kenkyusho) and DCA 2000 System (manufactured by Bayer Diagnosics), respectively. The results are expressed by the average ± standard error with respect to each group. The significant difference test as compared with the control group was performed by the Student t-test.

### (Result 1)

The changes of blood sugar level and HbAIc with respect to each of the group dosed with the compound of the present invention and the control group are specified in the following Table.

**Table 2**

| Blood sugar level (mg/dl) | Day 0 | Day 3 | Day 7 | Day 10 | Day 14 |
|---|---|---|---|---|---|
| invention compd. group | 465.7 ±21.51 | 380.8 ±52.42 | 319.8 ±27.49 | 334.3 ±33.75 | 280.2 ±37.31 |
| control group | 469.7 ±23.14 | 455.0 ±29.52 | 444.5 ±17.86 | 423.5 ±32.85 | 374.5 ±40.91 |

**Table 3**

| HbAIc (mg/dl) | Day 0 | Day 14 |
|---|---|---|
| invention compd. group | 12.5 ±0.35 | 9.4 ±0.69 |
| control group | 12.8 ±0.57 | 10.2 ±0.61 |

[Blood sugar level (mg/dl) of the group dosed with the compound of the present invention: 465.7 ± 21.51 on day 0, 380.8 ± 52.42 on day 3, 319.8 ± 27.49 on day 7, 334.3 ± 33.75 on day 10 and 280.2 ± 37.31 on day 14 / Blood sugar level (mg/dl) of the control group: 469.7 ± 23.14 on day 0, 455.0 ± 29.52 on day 3, 444.5 ± 17.86 on day 7, 423.5 ± 32.85 on day 10 and 374.5 ± 40.91 on day 14]

It is apparent from Table 2 and Fig. 1 that the compound of the present invention has excellent hypoglycemic effect.

As apparent from Table 3, with respect to HbAIc as well, a decrease trend is recognized in the group dosed with the compound of the present invention as compared with the control group.

### Toxicity Test Example

### (Experimental Method 2)

(E)-3-[2-(5,6-Dimethoxy-3-methyl-1,4-benzoquinonyl)]-2-nonylpropenoic acid was used as a test compound.

### (1) Rat:

250, 500, 1000 and 2000 mg/kg of the test compound were forcibly administered once to SD rat groups each consisting of three males and three females and they were observed for 14 days.

10, 20 and 60 (20 every hour × 3) mg/kg of the test compound were forcibly administered once to SD rat groups each consisting of five males and five females and they were observed for 14 days.

### (2) Beagle:

100, 200 and 400 mg/kg of the test compound were forcibly administered once to beagle groups each consisting of one male and one female and they were observed for 21 days.

### (Result 2)

The lethal dose to rat and beagle at single administration is given in the following Table.

**Table 4**

| Animal sp. | Route of admin. | Lethal dose (mg/Kg) |
|---|---|---|
| rat | p.o. | 2000 |
| | i.v. | >60 |
| beagle | p.o. | >400 |

The excellent safety of the compound of the present invention is apparent from Table 4.

## Claims

1. A method for lowering blood sugar or glucose, comprising administering a pharmacologically effective amount of a quinone derivative represented by the following general formula (I) or pharmacologically acceptable salts thereof to a person suffering from the hypoglycemic. 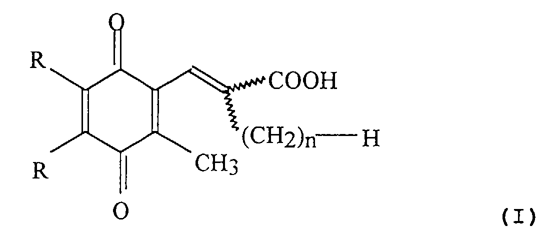 wherein n is 0 or an integer of 1 to 12 and R is a methyl or methoxy group.

2. The method claimed in Claim 1, wherein the quinone derivative (I) is (E)-3-[2-(5,6-dimethoxy-3-methyl-1,4-benzoquinonyl)]-2 -nonylpropenoic acid represented by the following chemical formula (II):

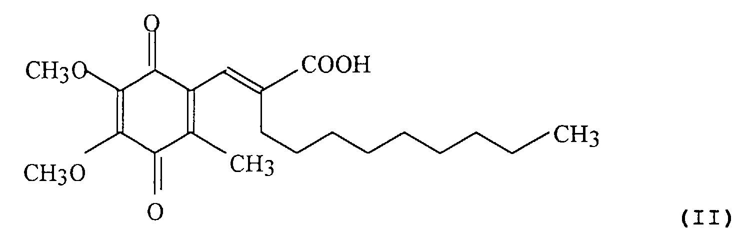

3. Use of a quinone derivative represented by the following general formula (I) or pharmacologically acceptable salts thereof for manufacturing a hypoglycemic agent: 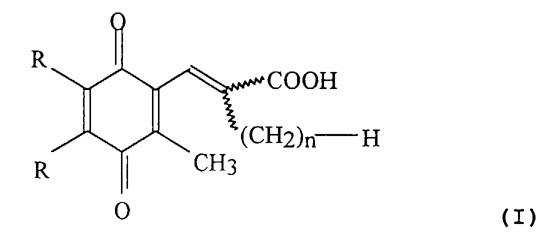 wherein n is 0 or an integer of 1 to 12 and R is a methyl or methoxy group.

4. A method for preventing, curing or ameliorating diabetes, comprising administering a pharmacologically effective amount of a quinone derivative represented by the following general formula (I) or pharmacologically acceptable salts thereof to a person suffering from the diabetes. 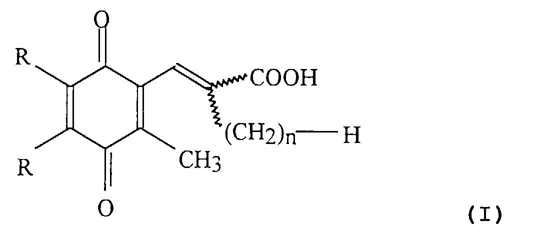 wherein n and R have the same meanings as mentioned above.

5. The method claimed in Claim 4, wherein the quinone derivative (I) is (E)-3-[2-(5,6-dimethoxy-3-methyl-1,4-benzoquinonyl)]-2-nonylpropenoic acid represented by the following chemical formula (II):

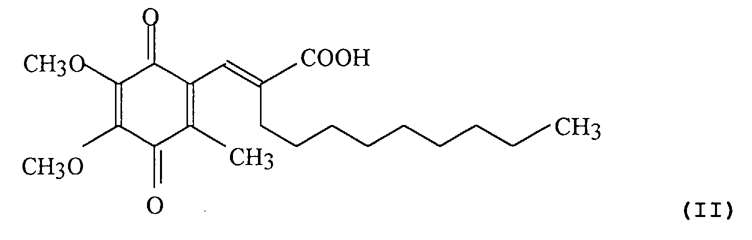

6. Use as claimed in Claim 3, in which the the hypoglycemic agent is a diabetes preventing, curing or ameliorating agent.
